# EUROPEAN PATENT APPLICATION

(11) **EP 4 376 261 A1**
(43) Date of publication of application: **29.05.2024**
(21) Application number: 22209624.0
(22) Date of filing: 25.11.2022
(51) Int. Cl.: H02J 50/10

(54) **A RECEIVER CIRCUITRY AND A METHOD FOR RECEIVING POWER THROUGH WIRELESS POWER TRANSFER, AND AN IMPLANT DEVICE**

(71) Applicant: Stichting IMEC Nederland, 5656 AE Eindhoven (NL)
(72) Inventor: Stanzione, Stefano, 5508 AH Veldhoven (NL); Vis, Peter, 5432 BX Cuijk (NL)
(74) Representative: AWA Sweden AB

(57) **Abstract**

A receiver circuitry (100) for receiving power through wireless power transfer comprises: a power receiver (102) configured to receive a power signal from a wireless power transmitter and generate an alternating current, AC, input signal (V_{AC}); a converter circuit (110), configured to receive the AC input signal (V_{AC}) and to convert the AC input signal (V_{AC}) to a direct current, DC, output signal (V_{OUT}); a control unit (140) configured to control at least one parameter of the converter circuit (110) for controlling efficiency of power transfer; a detector (130) configured to generate a measurement signal (Vₚₑₐₖ) representing available power of the AC input signal (V_{AC}); wherein the control unit (140) is configured to receive the measurement signal from the detector (130) and to control the at least one parameter based on the measurement signal for feedforward control of the at least one parameter.

## Description

### Technical field

The present description relates to a receiver circuitry and a method for receiving power through wireless power transfer. The present description further relates to an implant device comprising the receiver circuitry.

### Background

Power transfer is done in various applications. In many applications, such as in energy harvesting, where power is to be transferred from an energy source, maximum power point tracking (MPPT) algorithms are used in order to ensure that a high efficiency of the power transfer is provided. The MPPT algorithm may ensure that the power transfer is done at a point at which maximum power transfer is provided. This may imply that impedance matching between the energy source and a load is provided.

In wireless power transfer, it may be difficult to implement an MPPT algorithm. There may be switching losses and conduction losses based on powering control, which losses are a non-linear function of the power delivered to the load. Thus, in order to provide MPPT for the power transfer, it may be necessary to estimate output power to the load based on measurement of a load current. However, measurement of the load current may be complicated, in particular at low power, and the determination of impedance matching based on the measurement may in itself consume power.

Further, the MPPT algorithm may be relatively slow such that a maximum power point may not be reached in power transfer conditions that are varying relatively quickly. Thus, if wireless power transfer is performed in a dynamic environment, such as when providing wireless power transfer to an implanted device, the MPPT algorithm may never reach the maximum power point.

### Summary

An objective of the present description is to provide control of wireless power transfer, such that efficient power transfer is provided. A particular objective of the present description is to provide fast control of wireless power transfer, such that the wireless power transfer may be quickly adapted to changes in a dynamic environment for ensuring efficient power transfer even in varying conditions.

These and other objectives are at least partly met by the invention as defined in the independent claims. Preferred embodiments are set out in the dependent claims.

According to a first aspect, there is provided a receiver circuitry for receiving power through wireless power transfer, said receiver circuitry comprising: a power receiver configured to receive a power signal from a wireless power transmitter and generate an alternating current, AC, input signal; a converter circuit, configured to receive the AC input signal and to convert the AC input signal to a direct current, DC, output signal; a control unit configured to control at least one parameter of the converter circuit for controlling efficiency of power transfer; a detector configured to generate a measurement signal representing available power of the AC input signal; wherein the control unit is configured to receive the measurement signal from the detector and to control the at least one parameter based on the measurement signal for feedforward control of the at least one parameter.

The receiver circuitry is configured to provide a measurement representing available power of the AC input signal. The receiver circuitry further uses the measurement for feedforward control for controlling at least one parameter for controlling efficiency of the power transfer.

This implies that the receiver circuitry does not need to measure a load current and does not need to perform complicated determinations of the power output to a load. Rather, the receiver circuitry may provide a simple measurement of the available power of the AC input signal and use this as input for controlling the at least one parameter in a feedforward manner. The receiver circuitry may thus ensure that control of efficiency of power transfer is provided in a power efficient manner.

The at least one parameter of the converter circuit may be controlled so as to provide control of impedance in order to control efficiency of power transfer. Thus, the at least one parameter may be controlled in order to match source and load impedance. However, it should be realized that the control may not be able to perfectly match the source and load impedance, while the control still provides efficient power transfer.

The power receiver is configured to receive a wireless power signal. The power receiver may for instance be configured to receive an inductive power signal. Thus, the power receiver may comprise a receiver coil configured to receive the power signal from a transmitter coil. The power receiver may thus be configured to use the receiver coil, coupled to the transmitter coil with a coupling factor, a parasitic resistance of the power receiver and a resonance capacitance of the power receiver for generating the AC input signal based on the received power signal.'

The converter circuit is configured to convert the AC input signal to a DC output signal that may be provided to a load. The converter circuit may be configured to comprise a rectifier circuit for converting the AC input signal to a DC signal. The converter circuit may further comprise a DC-DC converter circuit for providing control of the DC output signal. However, it should be realized that the DC-DC converter may not be necessarily used. For instance, for transfer of high power, the DC-DC converter does not need to be used, even though the DC-DC converter may be preferred since it allows regulation of the output voltage to the load.

The control unit may be configured to control at least one parameter of the converter circuit that affects efficiency of power transfer. For instance, the control unit may be configured to control at least one parameter of the converter circuit affecting impedance matching between the power source and the load. The control unit may be configured to control one parameter which has a largest effect on impedance matching. The control unit may be configured to control more than one parameter such that a high degree of impedance matching is achieved. The control unit may for instance be configured to control at least one parameter of a rectifier circuit and/or a DC-DC converter circuit of the converter circuit.

The detector is configured to generate a measurement signal representing available power of the AC input signal. Thus, the receiver circuitry is configured to determine the available power rather than determine the actual power output to the load. This implies that a simple measurement may be performed, which may still be used by the control unit for controlling efficiency of power transfer in a feedforward manner.

The detector may be configured to generate the measurement signal based on detection of the AC input signal itself.

According to embodiments, the detector may be configured to generate the measurement signal based on measurement of output of a rectifier circuit. Such measurement may provide a representation of the available power of the AC input signal based on a known transfer characteristic of the rectifier circuit or based on simulations that are used in a look-up table for converting the measurement of output of the rectifier circuit to the available power of the AC input signal.

According to an embodiment, the detector is a peak detector configured to detect an amplitude of the AC input signal for the power receiver being unloaded, whereby the measurement signal represents the amplitude of the AC input signal.

The peak detector provides a simple manner of determining available power of the AC input signal, such that the measurement signal may be provided in a simple and power-efficient manner. Further, the control unit may be configured to directly use the measurement signal for controlling the at least one parameter.

The receiver circuitry may be configured to provide detection of the amplitude of the AC input signal when the power receiver is unloaded. This implies that the receiver circuitry may comprise a measurement switch for briefly disconnecting the power receiver from the converter circuit during detection of the amplitude of the AC input signal. Also, when the power receiver is unloaded, the rectifier is disabled or disconnected from input to the rectifier.

It should be realized that the receiver circuitry may be configured to detect the amplitude of the AC input signal while the power receiver is loaded. Thus, no measurement switch may be needed. This may be used if non-linear load is known or characterized such that the detected amplitude may be converted to a measurement signal representing available power of the AC input signal.

According to an embodiment, the detector comprises an active peak detector.

This may be useful if low amplitudes of the power signal are to be determined.

According to another embodiment, the detector comprises a passive peak detector.

A passive peak detector may consume less power than an active peak detector. Also, the passive peak detector may be a simpler component than the active peak detector. Therefore, the receiver circuitry may preferably comprise a passive peak detector.

However, the active peak detector may be able to detect lower amplitudes and may be preferred if low signal amplitudes are to be detected.

According to an embodiment, the control unit is configured to store a conversion rule for converting a value of the measurement signal to a control signal for controlling a value of the at least one parameter.

Thus, the control unit may be configured to determine a value of the at least one parameter based on a stored conversion rule. This implies that the control unit may directly determine the value of the at least one parameter based on the stored conversion rule. The determining of the value of the at least one parameter may thus be done in a fast and power efficient manner.

According to an embodiment, the conversion rule is based on simulation of the receiver circuitry.

Thus, the simulation of the receiver circuitry may characterize the receiver circuitry to provide the conversion rule. This implies that the conversion rule may be determined in a simple manner based on design of the receiver circuitry.

The conversion rule may also or alternatively be based on measurements on the receiver circuitry. Thus, one or more calibration measurements may be performed in order to determine the conversion rule. The calibration measurements may be performed for each manufactured receiver circuitry or for one or more receiver circuitry, wherein the calibration measurements are used for plural receiver circuitries. By using a few or no calibration measurements, a manufacturing process is minimally affected.

The calibration measurements may be used in combination with simulations. Thus, the calibration measurements do not need to be performed for completely characterizing proper conversion of a value of a measurement signal to a desired value of the at least one parameter. Rather, one or a few calibration points may be measured, which may be used for adjusting a conversion rule determined by simulation of the receiver circuitry.

According to an embodiment, the conversion rule is represented as a functional relationship between the value of the measurement signal and the value of the at least one parameter or as a look-up table.

Thus, the value of the at least one parameter may be determined very quickly, by computing the value through the functional relationship or by look-up in the look-up table.

According to an embodiment, wherein the converter circuit comprises a rectifier circuit, configured to receive the AC input signal and to convert the AC input signal to a DC input signal, and a DC-DC converter circuit configured to convert the DC input signal to the DC output signal, wherein the control unit is configured to control more than one parameter of the rectifier circuit and/or the DC-DC converter circuit.

By controlling more than one parameter of the converter circuit, the receiver circuitry may be configured to provide more accurate control of efficiency of power transfer. For instance, control of more than one parameter may allow load impedance to be more closely matched to source impedance.

Thanks to the use of feedforward control, the control unit may ensure that the receiver circuitry is immediately adapted to the available power of the AC input signal that is received. This implies that the control does not need to be iteratively improved for adapting the receiver circuitry to changing conditions for power transfer.

In comparison, if feedback control is provided, the receiver circuitry may not be able to be adapted to new conditions before the conditions are changed again. In particular when more than one parameter is controlled, the use of feedforward control is efficient in ensuring that an efficient power transfer is provided. However, it should be realized that also when only one parameter is controlled, the use of feedforward control may substantially improve possibility to reach a maximum power point compared to use of feedback control.

According to an embodiment, the rectifier circuit comprises a comparator configured to drive a switch.

Thus, the rectifier circuit may be an active rectifier. This may imply that the rectifier circuit has a low voltage drop so that the receiver circuitry is power efficient.

According to an embodiment, the at least one parameter comprises a size of the switch.

The rectifier circuit may comprise a switch comprising a plurality of switch components that may be turned on or off for providing a function of the switch. The switch components may be arranged in parallel such that by controlling a number of switch components that are turned on, a series resistance from input to output of the rectifier circuit may be controlled. Thus, the at least one parameter may control the size of the switch by determining the number of switch components that are involved in the function of the switch.

The size of the switch may be controlled to be small to ensure that switching losses are reduced when the available power of the AC input signal is low, because driving a gate of a large number of switch components consumes relatively large amount of power. Further, the size of the switch may be controlled to be large to ensure that conduction losses are reduced when the available power of the AC input signal is high.

According to an embodiment, the rectifier circuit further comprises a divider, wherein the at least one parameter comprises a divider value for controlling a number of oscillation periods of the input signal per extraction of power by the rectifier circuit.

In other words, the rectifier circuit may be controlled such that power is extracted once per a number *N* of oscillation periods. Thus, by controlling the divider value *N*, the rectifier circuit may be controlled to extract power once every *N* oscillation periods.

This may be used to allow accumulation of power when the available power of the AC input signal is low. Thus, a total efficiency of converting the AC input signal to the DC output signal may be controlled based on the divider value.

According to an embodiment, the at least one parameter comprises a reference voltage received by the DC-DC converter circuit.

The reference voltage may define an input voltage of the DC-DC converter circuit.

Changing of the reference voltage at a given level of the available power implies that the impedance of the converter circuit is changed. This implies that the impedance of the receiver circuitry may be changed to provide impedance matching between a source impedance and a load impedance.

According to a second aspect, there is provided an implant device comprising the receiver circuitry according to the first aspect, wherein the implant device is configured to be implanted in a body and configured to receive power through wireless power transfer.

Effects and features of this second aspect are largely analogous to those described above in connection with the first aspect. Embodiments mentioned in relation to the first aspect are largely compatible with the second aspect.

Wireless powering of an implant device may be of particular interest because it may enable substantially increasing lifetime of the implant device. For instance, using wireless power transfer, a battery of the implant device may be recharged. Alternatively, using wireless power transfer, the implant device may be directly powered.

The wireless power transfer to the implant device may be subject to dynamically changing conditions. For instance, the physical relation between a power transmitter arranged outside a body and the implant device may change over time, as the implant device may change position and/or orientation within the body. Thus, thanks to the use of the receiver circuitry according to the first aspect in the implant device of the second aspect, a high efficiency of the wireless power transfer may be ensured and maintained even in dynamically changing conditions of the wireless power transfer.

Thanks to an efficient power transfer being provided, a distance between the power transmitter and the receiver circuitry may not be as critical. The wireless power transfer may be sufficiently efficient even though the distance between the power transmitter outside the body and the receiver circuitry is not minimized. This allows for a flexible arrangement of the power transmitter in relation to the implant device.

The implant device may be configured to be implanted in a human body or an animal body.

According to a third aspect, there is provided a method for receiving power through wireless power transfer, said method comprising: receiving a power signal from a wireless power transmitter; generating an alternating current, AC, input signal based on the power signal; measuring the AC input signal to generate a measurement signal representing available power of the AC input signal; controlling at least one parameter based on the measurement signal for feedforward control of the at least one parameter, wherein the at least one parameter is at least one parameter of a converter circuit, configured to receive the AC input signal and to convert the AC input signal to a direct current, DC, output signal.

Effects and features of this third aspect are largely analogous to those described above in connection with the first and second aspects. Embodiments mentioned in relation to the first and second aspects are largely compatible with the third aspect.

Thanks to the feedforward control of the at least one parameter, the method may allow controlling the wireless power transfer to ensure efficient power transfer, even in changing conditions.

### Brief description of the drawings

The above, as well as additional objects, features, and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a receiver circuitry according to an embodiment.
Fig. 2 is a graph illustrating a relationship between a reference voltage of a DC-DC converter circuit of the receiver circuitry and a peak amplitude of an AC input signal.
Fig. 3 is a graph illustrating a relationship between a size of a switch of a rectifier circuit of the receiver circuitry and a peak amplitude of an AC input signal.
Fig. 4 is a graph illustrating a relationship between a divider value of a divider of the rectifier circuit of the receiver circuitry and a peak amplitude of an AC input signal.
Fig. 5 is a schematic view of an implanted device comprising the receiver circuitry.
Fig. 6 is a flow chart of a method.

### Detailed description

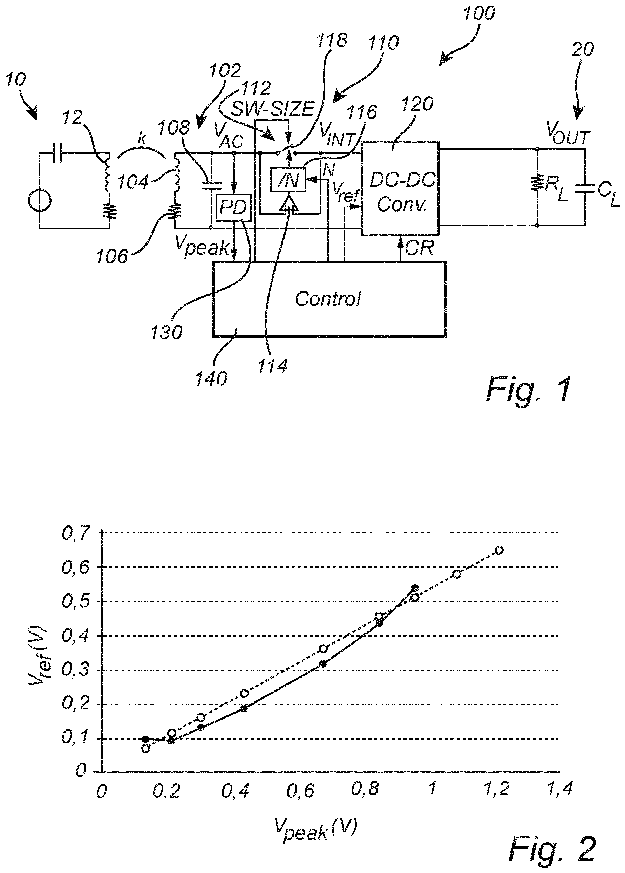

Referring now to Fig. 1, a power transmitter 10 and a receiver circuitry 100 for receiving power from the power transmitter 10 through wireless power transfer is shown.

The power transmitter 10 may comprise a coil 12 and may be configured to provide wireless power transfer through inductive coupling to a power receiver 102 of the receiver circuitry 100.

The power receiver 102 may comprise a coil 104 coupled to the coil of the power transmitter 10 with a coupling factor *k* and may further be represented by a parasitic resistance 106 and a resonance capacitance 108. The power receiver 102 may be configured to generate an alternating current (AC) input signal V_{AC} based on a powering signal received from the power transmitter 10.

The receiver circuitry 100 further comprises a converter circuit 110, which is configured to convert the AC input signal V_{AC} to a direct current (DC) signal. Control of the receiver circuitry 100 may cause power losses, such as switching losses and conduction losses in the converter circuit 110. Such power losses may be a non-linear function of the available power of the AC input signal V_{AC}.

In order to avoid power losses, power delivered to a load 20 by the receiver circuitry 100 could be measured. However, for low power systems, determining average output power may cause significant efficiency losses based on measurements that are needed. Estimation of output power may require averaging of a product of output voltage and load current, which is not simple at all. Measuring an average current is rather complicated at low power, because low energy losses in a measurement path can be achieved only using an electronic control (like an amplifier) with bandwidth sufficiently larger than a carrier frequency of the powering signal. When wireless power transfer is performed within a non-magnetic material (like air or an animal or human body), the carrier frequency is preferably in MHz range, such as larger than 1 MHz. This implies that power consumption of an amplifier in the measurement path will be large.

Further, control of the receiver circuitry 100 based on the average output power by the receiver circuitry 100 would provide a feedback system. The control may thus require a feedback loop for controlling one or more parameters of the receiver circuitry 100 in order to optimize loss reduction. This implies that the control may be slow as multiple iterations may be required in order to find optimal parameters of the receiver circuitry 100. Thus, in a dynamically changing environment, the control may not be able to follow changing conditions to optimize loss reduction and the wireless power transfer may be constantly inefficient.

As illustrated in Fig. 1, the receiver circuitry 100 comprises a detector 130 configured to generate a measurement signal representing available power of the AC input signal V_{AC}. This detector 130 is configured to provide the measurement signal to a control unit 140 configured to control at least one parameter of the converter circuit 110 for controlling efficiency of power transfer. Thus, the control unit 140 is configured to provide feedforward control of the at least one parameter, which implies that the control unit 140 may set a value of the at least one parameter such that the at least one parameter is immediately adapted to the available power of the AC input signal V_{AC}. Hence, the receiver circuitry 100 may be very quickly adapted to available power of the AC input signal V_{AC} such that losses of the receiver circuitry 100 may be reduced and the wireless power transfer may be provided in a very power efficient manner. The control of the at least one parameter by the control unit 140 may ensure that impedance matching between the load 20, including the receiver circuitry 100, and the power transmitter 10 may be achieved. This implies that the receiver circuitry 100 may be configured to provide maximum power point tracking (MPPT) for wireless power transfer in a dynamic environment.

The detector 130 may comprise a peak detector configured to detect a peak amplitude Vₚₑₐₖ of the AC input signal. The peak detector may be configured to detect the peak amplitude Vₚₑₐₖ when the power receiver 102 is unloaded. Thus, an open AC voltage may be detected by the peak detector.

The receiver circuitry 100 may comprise a switch disconnecting the power receiver 102 from the converter circuit 110 during detection of the peak amplitude of the AC input signal. However, if non-linear load of the converter circuit 110 is known or characterized, e.g., stored in a look-up table of the control unit 140, the receiver circuitry 100 may not need to disconnect the power receiver 102 from the converter circuit 110 during detection of the peak amplitude of the AC input signal.

The control unit 140 may be configured to periodically activate the switch for briefly disconnecting the power receiver 102 from the converter circuit 110. The detector 120 may be configured to detect the peak amplitude of the AC input signal while the power receiver 102 is briefly disconnected from the converter circuit 110. The control unit 140 may thus be configured to receive periodically updated measurement signals from the detector 130 and the control unit may be configured to periodically update control of the at least one parameter based on the measurement signal from the detector 130.

The detector 130 may comprise an active peak detector or a passive peak detector for detecting the peak amplitude of the AC input signal. A passive peak detector may consume less power than an active peak detector. Also, the passive peak detector may be a simpler component than the active peak detector. Thus, the passive peak detector may be preferred to provide a low power consumption for controlling efficiency of the power transfer. However, the active peak detector may be able to detect lower amplitudes and may be preferred if low signal amplitudes are to be detected.

The converter circuit 110 is configured to convert the AC input signal V_{AC} to a DC output signal V_{OUT} provided to the load 20. The converter circuit 110 may comprise a rectifier circuit 112, which is configured to receive the AC input signal V_{AC} and convert the AC input signal V_{AC} to a DC input signal V_{INT}, and a DC-DC converter 120, which is configured to convert the DC input signal V_{INT} to the DC output signal V_{OUT}.

The rectifier circuit 112 may be an active rectifier comprising a comparator 114 and a switch 118. The rectifier circuit 112 may be configured as a single wave rectifier but it should be realized that the rectifier circuit 112 may be configured in many different ways, such as a full wave rectifier.

The comparator 114 may be configured to sense voltage of the AC input signal V_{AC} and to control switch components of the switch 118 to be opened in dependence of the sensed voltage such that a current output by the rectifier circuit 112 is controlled to flow in correct direction.

The rectifier circuit 112 may comprise diodes and may be configured to passively provide conversion of the AC input signal to the DC input signal. However, diodes may be associated with a relatively large voltage drop such that the rectifier circuit 112 may cause significant power losses. Therefore, use of an active rectifier may be preferred.

The switch 118 may comprise a plurality of switch components that are controlled in order to provide flow of current in the correct direction from the rectifier circuit 112. A number of switch components being activated may be controlled in order to control a size of the switch 118. The size of the switch 118 may be associated with power losses through switching losses and conduction losses. Thus, the size of the switch 118 may preferably be small when the available power of the AC input signal V_{AC} is low in order to reduce switching losses, whereas the size of the switch 118 may preferably be larger when the available power of the AC input signal V_{AC} is high in order to reduce conduction losses.

The rectifier circuit 112 may further comprise a divider 116 such that the comparator 114 is followed by the divider 116. The divider 116 may be configured to control power extraction through the switch 118 such that the rectifier circuit 112 may be configured to extract power once every *N* oscillation periods of the AC input signal V_{AC}. Thus, the divider 116 may be configured based on a divider value to control when power is to be extracted such that a number of oscillation periods associated with each power extraction by the rectifier circuit 112 is controlled.

The divider 116 may thus allow accumulation of power during plural oscillation periods when power of the AC input signal V_{AC} is low. This implies that a total efficiency of converting the AC input signal V_{AC} to the DC output signal V_{OUT} may be dependent on the divider value.

The DC-DC converter circuit 120 may affect impedance matching. The DC-DC converter circuit 120 may be configured to impose the DC input voltage V_{INT} to be equal to a reference voltage V_{ref} received by the DC-DC converter circuit 120. The DC-DC converter circuit 120 may thus define an output impedance of the rectifier circuit 112. The reference voltage V_{ref} of the DC-DC converter circuit 120 may thus be controlled to control impedance matching and to control efficiency of power transfer.

The DC-DC converter circuit 120 may provide a conversion ratio defining a ratio between an output voltage V_{OUT} of the DC-DC converter circuit 120 and the reference voltage V_{ref}. In order for the DC-DC converter circuit 120 to provide a desired output voltage V_{OUT}, the conversion ratio CR may need to be changed in relation any change of the reference voltage V_{ref}. Thus, when the reference voltage V_{ref} is controlled, the conversion ratio CR may be changed in correspondence with a change of the reference voltage V_{ref} such that the desired output voltage is provided by the DC-DC converter circuit 120.

The control unit 140 may be configured to control at least one parameter of the converter circuit 110 for controlling efficiency of power transfer. For instance, the control unit 140 may be configured to control at least one of the switch size SW-SIZE of the rectifier circuit 112, the divider value N of the divider 116 of the rectifier circuit 112, or the reference voltage V_{ref} of the DC-DC converter circuit 120. It should be realized that the converter circuit 110 may be implemented in a different manner and that the control unit 140 may be configured to control other parameter(s) of the converter circuit 110.

The control unit 140 may be configured to control more than one parameter of the converter circuit 110. This may imply that efficiency of power transfer may be very accurately controlled.

The control unit 140 may have access to information of a relation between the available power of the AC input signal and suitable values of the at least one parameter to be controlled. The information may be based on a simulation of the receiver circuitry 100 and/or calibration measurements. Calibration measurements may be performed for a specimen of the receiver circuitry 100 and may be used for a plurality of receiver circuitries 100 such that calibrations need not be performed for each manufactured specimen. Calibration measurements may be performed based on one or a few values of the available power of the AC input signal and may be used for adapting information based on simulation to the actual receiver circuitry 100.

The control unit 140 may be configured to store in a non-volatile memory a conversion rule for converting a value of the measurement signal, representative of the available power of the AC input signal, to a desired value of the at least one parameter. The control unit 140 may further be configured to output a control signal to the converter circuit 110 for controlling the value of the at least one parameter.

The conversion rule may define a functional relationship, such that the desired value of the at least one parameter may be calculated using the functional relationship and the measurement of the available power of the AC input signal. The conversion rule may alternatively be provided as a look-up table, such that the desired value of the at least one parameter may be determined by a look-up operation in the look-up table using the measurement of the available power of the AC input signal as input to the look-up operation.

The control unit 140 may be implemented as software being executed on a processor, such as a microprocessor or a central processing unit (CPU), as firmware in an embedded system, or as hardware in form of a specifically designed processing unit, such as an Application-Specific Integrated Circuit (ASIC) or a Field-Programmable Gate Array (FPGA).

Referring now to Fig. 2, control of the reference voltage V_{ref} of the DC-DC converter circuit 120 as function of the peak amplitude Vₚₑₐₖ of the AC input signal is discussed.

Fig. 2 represents a graph of an example of an optimal load of the active rectifier 112, corresponding to the reference voltage V_{ref} of the DC-DC converter circuit 120 (which imposes the DC input voltage V_{INT} to be equal to the reference voltage V_{ref}), as function of the peak amplitude Vₚₑₐₖ of the AC input signal. The relation between the optimal load and the peak amplitude is represented as a solid curve in Fig. 2.

As shown in Fig. 2, the relation between V_{ref} and Vₚₑₐₖ may be approximated with a linear curve (dashed line in Fig. 2) without much error. A linear curve may preferably be used, since this would maintain a conduction angle constant while power level varies (which is a good condition to achieve close to optimal loading). Further, a linear approximation is very simple to implement and may imply that the control unit 140 may be able to determine the desired value of the reference voltage V_{ref} in a fast and power efficient manner.

However, it should be realized that a different polynomial interpolation for defining the functional relationship between V_{ref} and Vₚₑₐₖ is possible and may alternatively be used. The control unit 140 may thus store a functional relationship for allowing calculation of the desired value of V_{ref}. Alternatively, the control unit 140 may use a look-up table representing the relationship between V_{ref} and Vₚₑₐₖ.

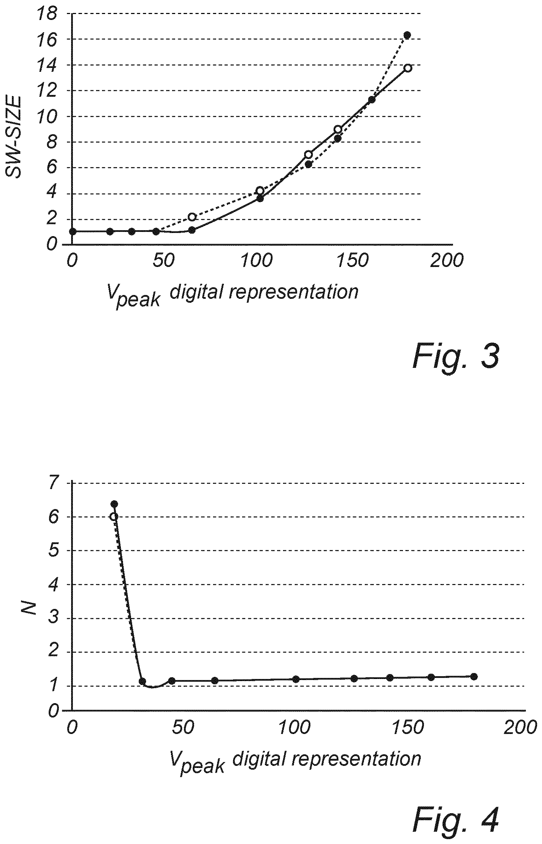

Referring now to Fig. 3, control of the size SW-SIZE of the switch 118 of the rectifier circuit 112 as function of the peak amplitude Vₚₑₐₖ of the AC input signal is discussed.

The peak amplitude Vₚₑₐₖ can be digitalized and its digital representation can be used to determine the optimal settings for the size SW-SIZE of the switch 118. Thus, the peak amplitude Vₚₑₐₖ may be converted to a digital value representing analog values of the peak amplitude. The parameter for the size SW-SIZE of the switch 118 may be a digital value corresponding to a number of switch components of the switch 118 to be activated, such that, using a digital representation of Vₚₑₐₖ can allow for more energy efficient determination of the parameter.

The control unit 140 may for instance use polynomial approximation techniques or saturating linear behavior logic for determining the desired value of the parameter SW-SIZE. Such determination of the desired value of the parameter may be very difficult to implement in a low power method using analog circuits.

Optimal settings, indicated by solid line in Fig. 3 may be approximated by a suitable polynomial approximation, indicated by dashed line in Fig. 3. The control unit 140 may thus store a functional relationship between SW-SIZE and (a digital representation of) Vₚₑₐₖ for allowing calculation of the desired value of SW-SIZE. Alternatively, the control unit 140 may use a look-up table representing the relationship between SW-SIZE and the digital representation of Vₚₑₐₖ.

Referring now to Fig. 4, control of the divider value *N* of the divider 116 of the rectifier circuit 112 as function of the peak amplitude Vₚₑₐₖ of the AC input signal is discussed.

As described above for the control of the size SW-SIZE of the switch 118, the peak amplitude Vₚₑₐₖ can be digitalized and its digital representation can be used to determine the optimal settings for the divider value *N* of the divider 116. Thus, the peak amplitude Vₚₑₐₖ may be converted to a digital value representing analog values of the peak amplitude. The parameter for the divider value of the divider 116 is a digital value, such that, using a digital representation of Vₚₑₐₖ can allow for more energy efficient determination of the parameter.

The control unit 140 may for instance use polynomial approximation techniques or saturating linear behavior logic for determining the desired value of the parameter *N*. Such determination of the desired value of the parameter may be very difficult to implement in a low power method using analog circuits.

Optimal settings, indicated by solid line in Fig. 4 may be approximated by a suitable polynomial approximation, indicated by dashed line in Fig. 4. The control unit 140 may thus store a functional relationship between the divider value *N* and (a digital representation of) Vₚₑₐₖ for allowing calculation of the desired value of *N*. Alternatively, the control unit 140 may use a look-up table representing the relationship between *N* and the digital representation of Vₚₑₐₖ.

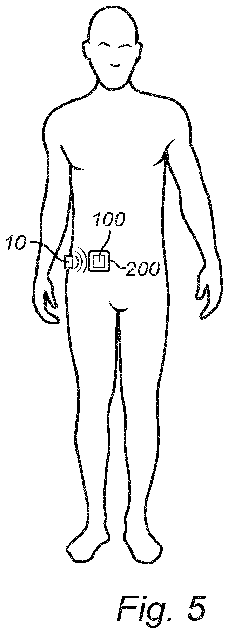

Referring now to Fig. 5, the receiver circuitry 100 may be advantageously provided in an implant device 200. Although it should be realized that the receiver circuitry 100 may be part of any device located in or inside the body, or on or outside the body, for use in any wireless power transfer regardless of where the device to be powered is arranged in relation to a power transmitter 10, the receiver circuitry 100 is particularly suited for use in the implant device 200.

The implant device 200 may be configured to be implanted in a human body or an animal body and may need wireless power transfer for powering the implant device 200 while being implanted. This implies that a relation between the implant device 200 and the power transmitter 10 may not be known or controlled. The wireless power transfer may be subject to dynamically changing conditions, for instance due to implant device 200 and the power transmitter 10 being subject to relative movement and/or orientation of the implant device 200 in relation to the power transmitter 10 being changed.

Thanks to the receiver circuitry 100, the wireless power transfer may be dynamically controlled in order to provide an efficient power transfer in the dynamic environment by the control unit 140 being configured to control the at least one parameter of the converter circuit 110.

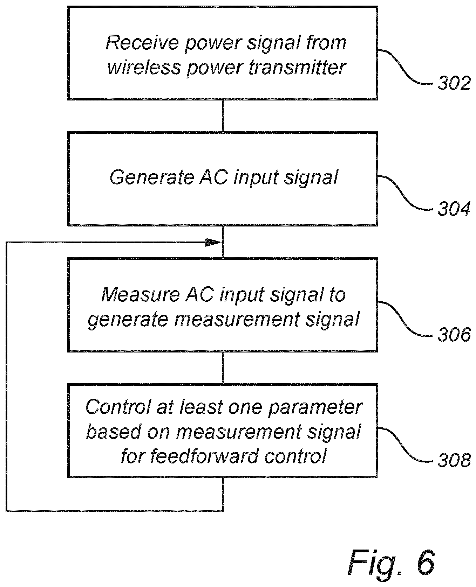

Referring now to Fig. 6, a method for receiving power through wireless power transfer will be described.

The method comprises receiving 302 a power signal from a wireless power transmitter. The power signal may be received by the power receiver 102 of the receiver circuitry 100.

The method further comprises generating 304 an AC input signal based on the power signal. The AC input signal may be generated by the power receiver 102.

The method further comprises measuring 306 the AC input signal to generate a measurement signal representing available power of the AC input signal. The AC input signal may be measured by the detector 130 configured to determine a peak voltage of the AC input signal.

The method further comprises controlling 308 at least one parameter based on the measurement signal for feedforward control of the at least one parameter, wherein the at least one parameter is at least one parameter of a converter circuit 110, configured to receive the AC input signal and to convert the AC input signal to a DC output signal. The controlling may be provided by a control unit 140 in dependence of the measurement signal received by the control unit 140. The control unit 140 may be configured, for instance, to control at least one of the switch size SW-SIZE of the rectifier circuit 112, the divider value N of the divider 116 of the rectifier circuit 112, or the reference voltage V_{ref} of the DC-DC converter circuit 120.

As illustrated in Fig. 6, the measuring 306 and controlling 308 may be iterated in order to continuously measure the AC input signal and control the at least one parameter for adapting the converter circuit 110 to changes in the peak voltage of the AC input signal.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A receiver circuitry (100) for receiving power through wireless power transfer, said receiver circuitry (100) comprising:
a power receiver (102) configured to receive a power signal from a wireless power transmitter and generate an alternating current, AC, input signal (V_{AC});
a converter circuit (110), configured to receive the AC input signal (V_{AC}) and to convert the AC input signal (V_{AC}) to a direct current, DC, output signal (VOUT);
a control unit (140) configured to control at least one parameter of the converter circuit (110) for controlling efficiency of power transfer;
a detector (130) configured to generate a measurement signal (Vₚₑₐₖ) representing available power of the AC input signal (V_{AC});
wherein the control unit (140) is configured to receive the measurement signal from the detector (130) and to control the at least one parameter based on the measurement signal for feedforward control of the at least one parameter.

2. The receiver circuitry according to claim 1, wherein the detector (130) is a peak detector configured to detect an amplitude of the AC input signal (V_{AC}) for the power receiver (102) being unloaded, whereby the measurement signal represents the amplitude of the AC input signal (V_{AC}).

3. The receiver circuitry according to claim 2, wherein the detector (130) comprises an active peak detector.

4. The receiver circuitry according to claim 2, wherein the detector (130) comprises a passive peak detector.

5. The receiver circuitry according to any one of the preceding claims, wherein the control unit (140) is configured to store a conversion rule for converting a value of the measurement signal to a control signal for controlling a value of the at least one parameter.

6. The receiver circuitry according to claim 5, wherein the conversion rule is based on simulation of the receiver circuitry.

7. The receiver circuitry according to claim 5 or 6, wherein the conversion rule is represented as a functional relationship between the value of the measurement signal and the value of the at least one parameter or as a look-up table.

8. The receiver circuitry according to any one of the preceding claims, wherein the converter circuit (110) comprises a rectifier circuit (112), configured to receive the AC input signal (V_{AC}) and to convert the AC input signal (V_{AC}) to a DC input signal (V_{INT}), and a DC-DC converter circuit (120) configured to convert the DC input signal (V_{INT}) to the DC output signal (V_{OUT}), wherein the control unit (140) is configured to control more than one parameter of the rectifier circuit (112) and/or the DC-DC converter circuit (120).

9. The receiver circuitry according to claim 8, wherein the rectifier circuit (112) comprises a comparator (114) configured to drive a switch (118).

10. The receiver circuitry according to claim 9, wherein the at least one parameter comprises a size of the switch (118).

11. The receiver circuitry according to claim 9 or 10, wherein the rectifier circuit (112) further comprises a divider (116), wherein the at least one parameter comprises a divider value for controlling a number of oscillation periods of the input signal per extraction of power by the rectifier circuit (112).

12. The receiver circuitry according to any one of claims 8-11, wherein the at least one parameter comprises a reference voltage received by the DC-DC converter circuit (120).

13. An implant device (200) comprising the receiver circuitry (100) according to any one of the preceding claims, wherein the implant device (200) is configured to be implanted in a body and configured to receive power through wireless power transfer.

14. A method for receiving power through wireless power transfer, said method comprising:
receiving (302) a power signal from a wireless power transmitter;
generating (304) an alternating current, AC, input signal based on the power signal;
measuring (306) the AC input signal to generate a measurement signal representing available power of the AC input signal;
controlling (308) at least one parameter based on the measurement signal for feedforward control of the at least one parameter, wherein the at least one parameter is at least one parameter of a converter circuit, configured to receive the AC input signal and to convert the AC input signal to a direct current, DC, output signal.
